Europäisches Patentamt

European Patent Office

Office européen des brevets

(11) Veröffentlichungsnummer: **0 267 386**

**A1**

(12) **EUROPÄISCHE PATENTANMELDUNG**

(21) Anmeldenummer: 87113254.4

(22) Anmeldetag: 10.09.87

(51) Int. Cl.⁴: **A61K 31/44** , A61K 9/22

(30) Priorität: 14.11.86 DE 3639073

(43) Veröffentlichungstag der Anmeldung:
18.05.88 Patentblatt 88/20

(84) Benannte Vertragsstaaten:
**AT BE CH DE ES FR GB GR IT LI LU NL SE**

(71) Anmelder: **Klinge Pharma GmbH**
**Berg-am-Laim-Strasse 129**
**D-8000 München 80(DE)**

(72) Erfinder: **Hofer, Josef-Maximilian**
**Bernauerstrasse 7**
**D-8018 Grafing(DE)**
Erfinder: **Knoch, Axel**
**Neumarkterstrasse 86d**
**D-8000 München 80(DE)**
Erfinder: **Stanislaus, Fritz**
**Halserspitzstrasse 12**
**D-8000 München 80(DE)**

(74) Vertreter: **Freiherr von Pechmann, Eckehart et**
**al**
**Patentanwälte Wuesthoff- v.**
**Pechmann-Behrens-Goetz Schweigerstrasse**
**2**
**D-8000 München 90(DE)**

(54) Feste Arzneizubereitungen und Verfahren zu ihrer Herstellung.

(57) Durch Verwendung niedrig viskoser Ethylcellulose, die zusammen mit Nifedipin auf Teilchen einer
Trägermasse aufgebracht ist, werden feste Arzneimittelzubereitungen mit verbesserter Wirkstofffreigabe erhalten.

EP 0 267 386 A1

## Feste Arzneizubereitungen und Verfahren zu ihrer Herstellung

Das 1,4-Dihydropyridinderivat Nifedipin zählt zur Klasse der Calciumantagonisten. Dieser Wirkstoff erweitert die Koronargefäße, senkt den peripheren Widerstand und verbessert dadurch die Sauerstoffversorgung des Myokards. Neben dem inzwischen klassichen Anwendungsbereich für Nifedipin, der belastungsabhängigen und der vasospastischen Angina pectoris, hat sich der Wirkstoff vor allem zur Behandlung des Bluthochdrucks bewährt. Voraussetzung für eine Therapie sind entsprechende Nifedipin-Plasmaspiegel. Zur erfolgreichen Behandlung des Bluthochdrucks sollen die Nifedipinspiegel zwischen 20 bis 80ng/ml liegen und bei Angina pectoris Spitzenwerte von 100 bis 200 ng/ml erreichen.

Aufgrund der schweren Wasserlöslichkeit von Nifedipin treten bei seiner galenischen Verarbeitung erhebliche Schwierigkeiten auf. Wie aus den zahlreichen Patentanmeldungen und Patentschriften für spezielle Formulierungen dieses Wirkstoffs hervorgeht, hat man bereits nach vielen verschiedenen Lösungswegen zur Herstellung brauchbarer Präparate gesucht. In den meisten Fällen liegt den Bemühungen die Schaffung einer schnell freisetzenden Arzneiform zugrunde. So beschreibt die DE-OS 28 22 882 feste, glasartige Kopräzipitate, die ein Gemisch aus Nifedipin und mindestens einem Hilfsstoff wie Polyvinylpyrrolidon oder einem Cellulosederivat darstellen. Auch die DE-OS 31 42 853 beschreibt feste Nifedipin-Zubereitungen, die aus einem Kopräzipitat von Nifedipin und Polyvinylpyrrolidon bestehen. In der DE-OS 34 13 643 werden Mischungen von Nifedipin und Zuckeralkoholen beansprucht. Die DE-OS 34 38 830 beschreibt eine Darreichungsform, in der das Nifedipin molekulardispers in einer erstarrten Schmelze in einer Mischung aus flüssigen und festen Polyethylenglykolen vorliegt.

Zur Therapie von Bluthochdruck und koronaren Herzerkrankungen benötigt man Arzneizubereitungen, die nicht nur zu rasch ansteigenden Plasmaspiegeln führen, sondern auch die notwendigen Wirkstoffkonzentrationen im Plasma innerhalb der therapeutisch empfohlenen Dosierintervalle aufrecht erhalten. Diese Voraussetzungen sind insofern schwierig zu erfüllen, da das Nifedipin eine relativ kurze Eliminationshalbwertzeit besitzt. Mit üblichen, schnell freisetzenden Arzneiformen lassen sich die notwendigen Nifedipin-Plasmaspiegel nicht über den geforderten Zeitraum aufrechterhalten, so daß eine zweckmäßige Therapie des Bluthochdrucks und der koronaren Herzerkrankungen nicht durchgeführt werden kann.

Inzwischen sind verschiedene Lösungswege beschrieben worden. In der EP-PS 0 047 899 werden Arzneizubereitungen angegeben, die nicht nur zu einem raschen Anstieg des Nifedipinspiegels führen, sondern auch die Plasmakonzentrationen über viele Stunden auf einem hohen Wert halten sollen. Dem Prinzip liegt zugrunde, daß Nifedipin-Kristalle unterschiedlicher Größe kombiniert werden. Dabei lösen sich kleinere Nifedipin-Kristalle - schneller auf, wodurch der Plasmaspiegel rasch ansteigt. Da der Lösungsvorgang bei den größeren Kristallen langsamer vor sich geht, kann die Plasmakonzentration über Stunden auf einem höheren Wert gehalten werden. Das in der Patentschrift beschriebene Verfahren hat den Nachteil, daß bei Mahlung mit Stift-oder Hammermühlen die Nifedipin-Kristalle nicht in der gewünschten, breiten Korngrößenverteilung anfallen. So läßt sich z.B. mit einer Stiftmühle auch bei niedriger Drehzahl nur eine relativ schmale Korngrößenverteilung erzielen, wobei das Material oft zu mehr als 95% kleiner als 50 µm anfällt. Um das in der EP-PS 0 047 899 geforderte Material zu erhalten, ist es notwendig, das Nifedipin-Mahlgut zunächst auszusieben und anschließend entsprechende Mengen der geforderten Kornfraktion zu vermischen. Dieses Verfahren ist sehr aufwendig, zumal die gesamten Vorgänge aufgrund der hohen Lichtinstabilität der Substanz unter Lichtschutz durchgeführt werden müssen. Auch ist von Nachteil, daß die Steuerung der Wirkstoff-Freisetzung ausschließlich über die Auflösung der Nifedipin-Kristalle geschieht, die wiederum von den jeweiligen individuellen Magen- und Darmverhältnissen, u.a. von der inter-und intraindividuellen Magenmotilität abhängt. Dem Fachmann ist bekannt, daß Arzneizubereitungen vorzuziehen sind, aus denen der Wirkstoff unabhängig von physiologischen Gegebenheiten des Magen-Darmtraktes freigesetzt wird.

A. Hasegawa et al [Chem. Pharm. Bull. 33, 1615-1619 (1985)] beschreiben eine Nifedipin-Retardzubereitung auf Basis der Einbettung von Nifedipin in polymere Hilfsstoffe, die sich im - schwach sauren Bereich des Magen-Darmtraktes auflösen. Im Vergleich zu einem im Handel befindlichen Nifedipin-Retardpräparat zeichnet sich die beschriebene Arzneizubereitung bei günstigen pharmakokinetischen Parametern hinsichtlich Retardierungseffekte durch eine verminderte Bioverfügbarkeit aus.

N. Kohri et al [J. Pharmac. Sci. 75, 57-61 (1986)] beschreiben Nifedipin-Retardzubereitungen unter Verwendung von Ethylcellulose mit einer Viskosität von 100 cP. Dabei wird ein Gew.-Teil Nifedipin und 1,5 bis 4,5 Gew.-Teile Ethylcellulose in Ethanol/Dichlormethan (1:1) gelöst und die Lösung in 5 bis 29 Gew.-Teile Stärke eingeteigt. Anschließend wird das Lösungsmittel entfernt und die Paste desagglomeriert. Von Nachteil erweist sich die Verwendung von erheblichen Mengen gesundheitsschädlichem Chlorkohlenwasserstoff. Vor allem ist dessen vollständige Entfernung im Produktionsmaßstab nur mit großem Aufwand möglich. Auch die übrigen beschriebenen Herstellschritte lassen sich im Produktionsmaßstab nur - schwer nachvollziehen. Außerdem zeigte sich bei der Nacharbeitung, daß mit diesem Verfahren nur ein geringer Teil des vorhandenen Nifedipins innerhalb der Verweildauer der oral applizierten Zubereitung im Magen-/Darmtrakt freigesetzt und resorbiert wird.

Überraschenderweise wurde festgestellt, daß durch Verwendung relativ geringer Mengen niedrig viskoser Ethylcellulose Nifedipin-Zubereitungen hergestellt werden können, bei denen der Wirkstoff im wesentlichen vollständig freigesetzt wird, so daß neben hoher Bioverfügbarkeit auch ausgezeichnete pharamkokinetische Parameter hinsichtlich Retardierungsstärke gesichert sind. Das bedeutet, daß das Nifedipin bereits kurz nach der Applikation in ausreichender Menge freigesetzt und dann doch eine anhaltende gleichmäßige Freigabe und Resorption zur Aufrechterhaltung des gewünschten Wirkstoffspiegels erreicht wird.

Die Erfindung betrifft daher neue Arzneizubereitungen in fester Form mit verbesserter Bioverfigbarkeit und Sicherstellung langanhaltender Nifedipin-Plasmaspiegel, die erfindungsgemäß unter Verwendung geringer Mengen niedrig viskoser Ethylcellulose mit einem Viskositätswert von 2 - 50 cP (mittleres Molekulargewicht 5000 - 20000) erhalten werden und bei denen das Nifedipin sich zusammen mit der Ethylcellulose auf Teilchen einer Trägermasse aufgebracht befindet. Dieses Material kann als solches oder zu Tabletten bzw. Dragees oder in Kapseln verarbeitet appliziert werden.

Bei den neuen Nifedipin-Zubereitungen soll das Gewichtsverhältnis von Nifedipin zur niedrigviskosen Ethylcellulose vorzugsweise im Bereich von 1 : 0,1 bis 1 : 1 liegen. Hinsichtlich der Trägermasse soll der Zuckeranteil, bezogen auf das Nifedipin, im Bereich von 1 : 1 bis 8 : 1 liegen, während der Hydroxyalkylcelluloseanteil 0,4 : 1 bis 4 : 1 und der Stärkeanteil 0,5 : 1 bis 5 : 1, jeweils bezogen auf die Nifedipinmenge, betragen soll.

Ein geeignetes Verfahren zur Herstellung der Zubereitungen besteht darin, daß man 1 Gew.-Teil reines Nifedipin zusammen mit 0,1 bis 1,0 Gew.-Teilen niedrig viskoser Ethylcellulose in 2 - 10 Gew.-Teilen eines für diese Mischung geeigneten organischen Lösungsmittels, z.B. Ethanol, löst und dann mit dieser Lösung eine Mischung aus 1 - 8 Gew.-Teilen Zucker, 0,4 - 4 Gew.-Teilen Hydroxyalkylcellulose und 0,5 - 5 Gew.-Teilen Stärke in einem Mischer befeuchtet. Die erhaltene Masse wird dann in üblicher Weise getrocknet und das Pulver bzw. das Granulat zur entsprechenden Arzneiform verarbeitet, z.B. zu Pellets, Tabletten oder Dragees oder es in Kapseln oder Sachets abgefüllt.

Die neuen Arzneizubereitungen stellen einen deutlichen Fortschritt in der Behandlung koronarer Herzkrankheiten und der Hypertonie dar. Darüberhinaus erlaubt das der Erfindung zugrunde liegende Herstellungsverfahren eine einfache, im Produktionsmaßstab zu realisierende Herstellung der Arzneizubereitungen ohne Komplikationen wegen schwierig zu handhabender Lösungsmittel.

Die nachfolgenden Ausführungsbeispiele erläutern die Erfindung. Hierbei ist zu beachten, daß wegen der hohen Lichtempfindlichkeit des Wirkstoffs Nifedipin alle Arbeiten unter Lichtschutz auszuführen sind. Die Viskositätswerte in cP für die jeweilige Ethylcellulose wurden gemäß National Formulary XVI mit einer 5%igen Toluol/Ethanol-(80/20)-Lösung bei 25°C bestimmt.

Beispiel 1

140 g Nifedipin und 70 g Ethylcellulose der Viskosität 7 cP werden in Ethanol in der Wärme klar gelöst. Sodann wird mit dieser Lösung eine Mischung aus 1.4 kg Saccharose, 600 g Maisstärke und 100 g Hydroxypropylmethylcellulose (4000 cP) in einem Mischer befeuchtet und 5 Minuten geknetet.

Nach 15-minütigem Trocknen bei 50°C wird durch ein Sieb der Maschenweite 0,50 mm desagglomeriert und erneut 15 Minuten bei 50°C getrocknet. Anschließend wird das 0,25 mm unterschreitende Korn abgesiebt und eingemahlen (= Unterkorn).

1,3 kg der Gutkornfraktion zwischen 0,25 und 0,50 mm werden denn in einem Dragierkessel vorgelegt, alternierend mit einer Lösung von 50 g Hydroxypropylmethylcellulose (4000 cP) in 1,2 kg Isopropanol befeuchtet und mit 0,8 kg der eingemahlenen Unterkornfraktion abgepudert (= Auftragsmischung). Nach Aufbringung der gesamten Auftragsmischung werden die Pellets zwischen 0,646 und 0,790 mm abgesiebt. Die diese Grenze über-oder unterschreitenden Pellets werden verworfen.

Die in den Grenzen liegenden Pellets werden im Umluft-Trockenschrank 12 Stunden bei 45°C nachgetrocknet und in HartgelatineKapseln der Größe 1 abgefüllt.

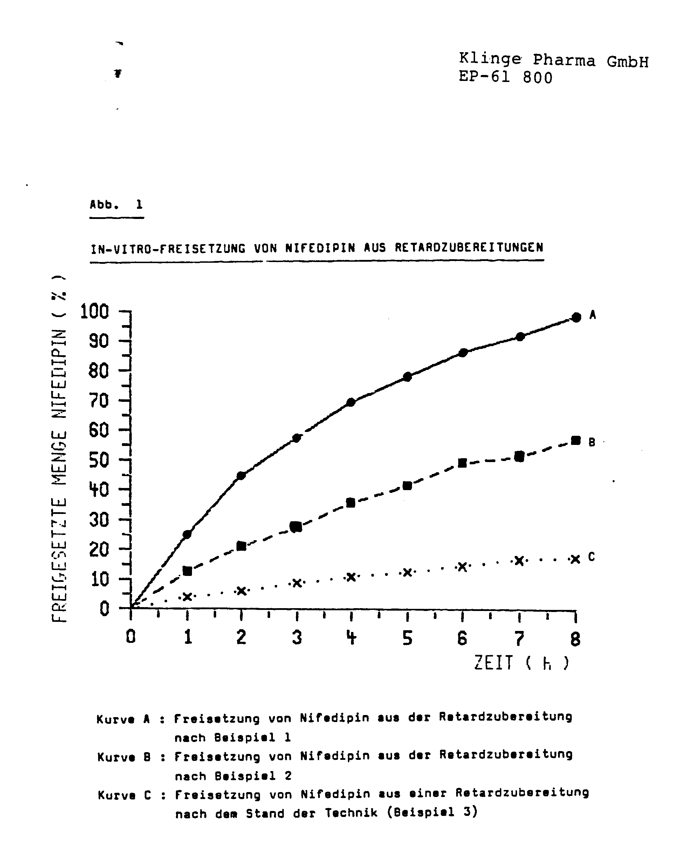

Die Untersuchung der Freigabe zeigt, wie die Kurve A in der Abbildung 1 erkennen läßt, eine gleichmäßige und vollständige in-vitro-Wirkstoff-Freisetzung von Nifedipin, bestimmt nach der Durchflußmethode (Flußgeschwindigkeit: 16,6 ml/min, 0,1 N HCl).

Beispiel 2

140 g Nifedipin und 100 g Ethylcellulose (7 cP) werden wie in Beispiel 1 in Ethanol klar gelöst und weiterverarbeitet.

Die Kurve B in der Abbildung 1 gibt die in-vitro-Freisetzung für eine erfindungsgemäße Zubereitung wieder, die gemäß Beispiel 2 hergestellt wurde, wobei die Freisetzung mit einer etwas größeren Verzögerung eintritt.

Vergleichsbeispiel

Um den Fortschritt der Erfindung aufzuzeigen, wurde eine Arzneizubereitung nach dem bisherigen Stand der Technik wie folgt hergestellt:

140 g Nifedipin und 100 g hochviskose Ethylcellulose (100 cP) werden in der Wärme in Ethanol/Dichlormethan (1:1) klar gelöst. Sodann wird mit dieser Lösung eine Mischung aus 1,8 kg Dicalciumphosphat und 120 g Hydroxypropylmethylcellulose (4000 cP) in einem Mischer befeuchtet, 7,5 Minuten geknetet und wie im Beispiel 1 weiter verarbeitet.

Die in-vitro-Freisetzung der so erhaltenen Zubereitung ist durch die Kurve C in der Abbildung 1 gezeigt. Während der üblichen Verweildauer einer oralen Applikationsform im Organismus von ca. 8 Stunden wird bei dieser vorbekannten Zubereitung nur maximal ca. 15% freigesetzt.

Es war nicht zu erwarten, daß allein durch die Wahl einer Ethylcellulose mit einem niedrigeren Viskositätswert ein so drastischer Unterschied in der Freigabe des Nifedipins erreichbar ist.

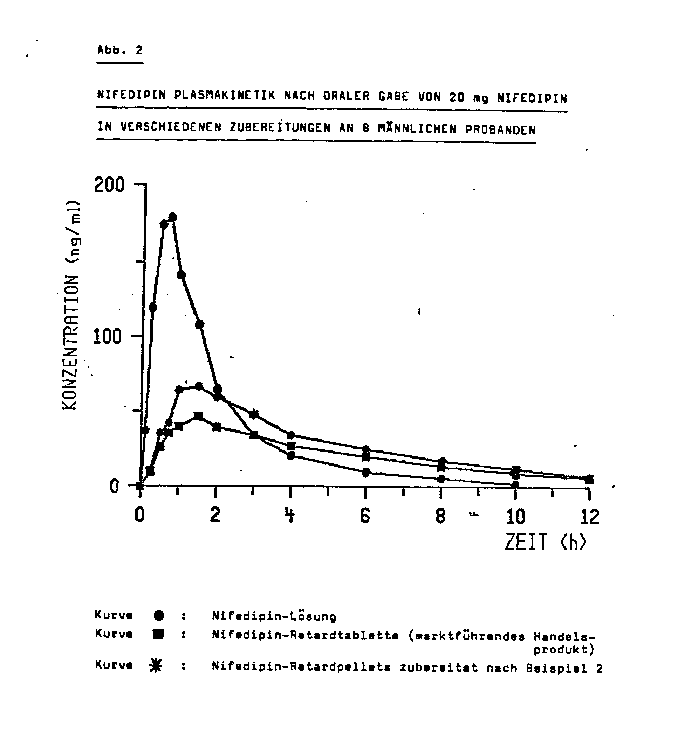

Auch bei therapeutischer Anwendung der erfindungsgemäßen Zubereitung war eine deutlich verbesserte Wirkung nachweisbar. Es wurde die Nifedipin-Plasmakinetik nach oraler Gabe bei jeweils 8 Personen im randomisierten cross-over-Versuch über mehrere Stunden nach der Applikation untersucht. Bei einer gemäß Beispiel 2 hergestellten Arzneiform ist die Plasmakonzentration im Vergleich zu einer Nifedipin-Lösung und einer im Handel befindlichen Retardtablette, die das Nifedipin in fein kristalliner Form enthält, hinsichtlich des Punktes Bioverfügbarkeit deutlich überlegen, bei ansonsten gleichen Parametern für die Stärke der Retardierung. Auch läßt die Abbildung 2 deutlich erkennen, daß die erfindungsgemäße Arzneizubereitung den Erhalt von therapeutisch notwendigen Plasmaspiegeln länger sicherstellt als die im Handel befindliche Retardtablette.

**Ansprüche**

1. Feste Arzneizubereitungen, bei denen das Nifedipin mit Ethylcellulose auf Teilchen einer Trägermasse aus Hydroxyalkylcellulose, Zucker und Stärke aufgebracht ist, dadurch **gekennzeichnet,** daß die Ethylcellulose eine niedrigviskose mit einem Viskositätswert von 2 - 50 cP ist.

2. Feste Arzneizubereitungen nach Anspruch 1, dadurch **gekennzeichnet,** daß das Gewichtsverhältnis von Nifedipin zu Ethylcellulose im Bereich von 1 : 0,1 bis 1 : 1 liegt.

3. Feste Arzneizubereitungen nach Anspruch 1 oder 2, dadurch **gekennzeichnet,** daß in der Trägermasse der Anteil von Zucker im Bereich von 1 : 1 bis 8 : 1, der von Hydroxyalkylcellulose im Bereich von 0,4 : 1 bis 4 : 1 und der von Stärke im Bereich von 0,5 1 bis 5 : 1, bezogen auf die Nifedipinmenge, liegt.

4. Feste Arzneizubereitungen nach Anspruch 1 bis 3, dadurch **gekennzeichnet,** daß sie zu Pulver, Granulat, Pellets, Tabletten, Dragees, Kapseln oder Sachets verarbeitet worden sind.

5. Verfahren zur Herstellung von festen Arzneizubereitungen nach Anspruch 1 bis 4, dadurch **gekennzeichnet,** daß man 1 Gew.-Teil Nifedipin und 0,1 bis 1,0 Gew.-Teile der niedrigviskosen Ethylcellulose in 2 bis 10 Gew.-Teilen organischem Lösungsmittel auflöst, mit der Lösung eine Mischung aus 1 bis 8 Gew.-Teilen Zucker, 0,4 bis 4 Gew.-Teilen Hydroxyalkylcellulose und 0,5 bis 5 Gew.-Teilen Stärke befeuchtet und die erhaltene Masse nach üblichen Methoden zu den Dosierungsformen weiter verarbeitet.

Klinge Pharma GmbH
EP-61 800

**Abb. 1**

IN-VITRO-FREISETZUNG VON NIFEDIPIN AUS RETARDZUBEREITUNGEN

Kurve A : Freisetzung von Nifedipin aus der Retardzubereitung
          nach Beispiel 1
Kurve B : Freisetzung von Nifedipin aus der Retardzubereitung
          nach Beispiel 2
Kurve C : Freisetzung von Nifedipin aus einer Retardzubereitung
          nach dem Stand der Technik (Beispiel 3)

Abb. 2

NIFEDIPIN PLASMAKINETIK NACH ORALER GABE VON 20 mg NIFEDIPIN

IN VERSCHIEDENEN ZUBEREITUNGEN AN 8 MÄNNLICHEN PROBANDEN

Kurve  ● :  Nifedipin-Lösung

Kurve  ■ :  Nifedipin-Retardtablette (marktführendes Handelsprodukt)

Kurve  ✳ :  Nifedipin-Retardpellets zubereitet nach Beispiel 2

| | **EINSCHLÄGIGE DOKUMENTE** | | EP 87113254.4 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| D,X | JOURNAL OF PHARMACEUTICAL SCIENCES, Band 75, Nr. 1, Jänner 1986, American Pharmaceutical Association<br><br>N. KOHRI et al. "Sustained Release of Nifedipine from Granules" Seiten 57-61<br><br>  * Seite 57, rechte Spalte "Preparation of Granules with pH-Independant Release", Tabelle I, Formulierungen D-H; Seite 58, rechte Spalte, Abb. 3,4 *<br><br>-- | 1,4,5 | A 61 K 31/44<br>A 61 K 9/22 |
| A | US - A - 4 369 172 (J.M. SCHOR et al.)<br><br>  * Zusammenfassung; Patentansprüche 1,2,18; Spalte 7, Zeilen 22-43 *<br><br>-- | 1-4 | |
| | | | **RECHERCHIERTE SACHGEBIETE (Int Cl 4)** |
| A | PATENT ABSTRACTS OF JAPAN, unexamined applications, Sektion C, Band 7, Nr. 209, 14. September 1983<br><br>THE PATENT OFFICE JAPANESE GOVERNMENT Seite 105 C 186<br><br>  * Kokai-Nr. 58-109 412 (TOUA EI YOU KAGAKU KOGYO K.K.) *<br><br>---- | 1-5 | A 61 K 31/00<br>A 61 K 9/00 |

Der vorliegende Recherchenbericht wurde für alle Patentansprüche erstellt.

| Recherchenort | Abschlußdatum der Recherche | Prüfer |
|---|---|---|
| WIEN | 12-02-1988 | MAZZUCCO |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82